# EUROPEAN PATENT SPECIFICATION

(11) **EP 4 103 139 B1**
(45) Date of publication and mention of the grant of the patent: **04.11.2026**
(21) Application number: 21710057.7
(22) Date of filing: 12.02.2021
(51) Int. Cl.: A61K 8/02, D01D 5/00, A61K 8/73, A61K 8/65, A61Q 19/08, B32B 5/02, B32B 5/24, B32B 5/26, B32B 15/14, B32B 15/20, B32B 27/36, D01F 1/10, D01F 2/28, D01F 4/00, D01F 9/00, A61Q 19/00, B32B 27/12

(54) **COSMETIC PRODUCT**
KOSMETISCHES PRODUKT
PRODUIT COSMÉTIQUE

(30) Priority: 13.02.2020 IT 202000002836
(43) Date of publication of application: 21.12.2022
(73) Proprietor: Bakel S.P.A., 33100 Udine (IT)
(72) Inventor: GREGORIS, Raffaella, 33100 Udine (IT); MANFREDINI, Stefano, 44049 Vigarano Mainarda (IT); VERTUANI, Silvia, 44123 Ferrara (IT); ROSO, Martina, 37142 Verona (IT)
(74) Representative: Petraz, Gilberto Luigi
(86) International application number: PCT/IB2021/051191
(87) International publication number: WO 2021/161251

(56) References cited:
- CN-A- 109 998 932
- GB-A- 2 484 319
- US-A1- 2015 272 855
- US-A1- 2017 251 789

## Description

### FIELD OF THE INVENTION

The present invention concerns a cosmetic product. More particularly, the invention concerns a cosmetic patch product that can be absorbed by the skin and based on polymer compounds, preferably biocompatible, consisting of fibers, in particular nanofibers, preferably obtained by electrospinning, and packaged.

### BACKGROUND OF THE INVENTION

Cosmetic products to be applied directly on the skin are widely known, which are formulated in various forms compatible with the skin and/or which are absorbed by the skin, such as for example, emulsions, creams, lotions, serums, gels, powders, oils, sun milks and suchlike, or other formulations.

These products, however, can sometimes be difficult to apply on the skin, sometimes requiring prolonged application times or a prolonged massage, in order to be completely absorbed into the dermis, and sometimes leaving cosmetic residues on the fingers or hands or on any possible applicators used.

There are also cosmetic products that are applied by means of a physical support, generally fibrous, which facilitates their application on the skin, such as in particular make-up remover wipes, which are imbued with a special cleansing composition.

One disadvantage of this type of cosmetic product is that, after use, the fibrous physical support, which does not have its own cosmetic function, as well as the packaging, have to be disposed of.

Furthermore, known fibrous physical supports have a specific surface that does not allow suitable transport and release of the active ingredients.

The question also arises that, generally, cosmetic compositions for the skin, for example those to be spread, such as cream, or in general cosmetic pastes, provide to use functional compounds for the skin and compounds that are exclusively functional for the structure of the formulation.

Functional compounds for the skin are partly or completely absorbed by the skin and bring benefits to the treated parts of the body. In general, this type of compound represents the so-called active ingredients.

The compounds that are exclusively functional for the structure of the formulation contribute to obtaining the cosmetic composition, whether it be an emulsion, a serum, or fluid, an oleolyte, a water, or a gel.

The effects of a compound that is exclusively functional for the structure of the formulation may affect the storage method, or the consistency of the cosmetic composition before its use and/or at the time of use, therefore when it is spread on the skin to be treated.

For example, a compound that is exclusively functional for the structure of the formulation can make the cosmetic composition soft, fluid or viscous; it allows to obtain a gel, prevent the separation of the oily compounds from the aqueous ones in an emulsion. Furthermore, compounds that are exclusively functional for the structure of the formulation allow to preserve the cosmetic composition, especially in the presence of water.

However, the problem arises that numerous compounds used, since they are functional for the structure of the formulation, do not bring any benefit to the skin, and indeed can even lead to a further deterioration of the treated skin. In fact, some of the compounds that are exclusively functional for the structure of the formulation remain indifferent to the skin, or they can cause, for example, further drying, pore closure, or development of allergies, dermatitis, or suchlike.

Among the compounds that are exclusively functional for the structure of the formulation, we can identify, for example, silicones, petrolatums (for example paraffin), alcohols, perfumes, stabilizers, preservatives, emulsifiers, the main function of which is to give the formulation of the cosmetic composition a "silk effect" consistency in contact with the skin and/or to give stability to the oily parts, or to increase the viscosity of gel formulations and/or increase the emollient and humectant effect of the emulsions.

For example, one problem that arises with these compounds exclusively functional for the structure of the formulation is the lack of skin compatibility, preventing the skin from breathing, also leading, for example, to its progressive drying, pore closure and/or greater sensitization.

It is also known that the presence of alcohols and preservatives on the one hand allow to preserve the cosmetic composition, and on the other hand increase the sensitization of the skin in developing dermatitis, allergies, or similar pathologies.

US 2015/272855 describes a cosmetic product in the form of a sheet, consisting of nanofibers obtained by electrospinning a solution comprising a water-soluble polymer, in particular polyvinyl alcohol PVA and/or polyvinylpyrrolidone PVP, and a functional material in a solvent of water and/or alcohol. The sheet product is intended to be applied to the skin for a prolonged time, typically for hours.

US2017/251789 shows a sheet-shaped cosmetic product consisting of nanofibers of water-soluble polymer material obtained by electrospinning a corresponding solution to be electrospun. The electrospun polymer material is mainly polyvinyl alcohol PVA and/or polyvinylpyrrolidone PVP. The solution also comprises a functional compound, in particular a cross-linking agent, which can be an acid, an alcohol or a derivative of formaldehyde. This sheet product is also intended to be left to act on the skin for a prolonged time.

GB2484319 on the contrary describes electrospun fibers based on honey and a biocompatible polymer, which can for example comprise a polysaccharide or a synthetic polymer such as polyethylene oxide PEO, polyethylene terephthalate PET or polypropylene PP. The fibers described have an antimicrobial effect and can be used to treat skin wounds.

There is therefore a need to perfect a cosmetic product that can overcome at least one of the disadvantages of the state of the art.

In particular, one purpose of the present invention is to provide a cosmetic product that can be applied simply and quickly to the skin.

Another purpose of the present invention is to provide a cosmetic product that does not have a physical support to be discarded after use.

Another purpose is to provide a cosmetic product which has a specific surface such as to optimize the transport and release of active ingredients, if there are any.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes, a cosmetic patch product has been produced which overcomes the limits of the state of the art and eliminates the defects present therein.

According to the invention, in particular, a cosmetic patch product is provided, to be applied preferentially on the skin, comprising a membrane substrate suitable to be absorbed by the skin. The membrane substrate is electrospun. The electrospun substrate is made up of fibers, preferably nanofibers, in particular in the form of non-woven fabric. The fibers are made up of hyaluronic acid and an electrospinning promoter selected from pullulan or a mixture of alginate with poly(oxyethylene).

Advantageously, the membrane substrate contains one or more active ingredients, for example peptides, amino acids, antioxidants or vitamins. With the expression "active ingredients" we mean, here and in this description, compounds that are functional for the skin that are absorbed in part, or completely, by the skin and bring benefits to the parts of the body being treated.

Preferably, the membrane substrate comprises linear and/or cross-linked hyaluronic acid. Advantageously, the membrane substrate is made both with linear hyaluronic acid and also with cross-linked hyaluronic acid.

Preferably, the hyaluronic acid has a molecular weight of less than 10,000 Da. This molecular weight is advantageous for the complete absorbability of the hyaluronic acid in the skin.

One advantage of the cosmetic patch product according to the embodiments described here is that it is completely absorbed by the skin, together with any active ingredient present, for example a cosmetic active ingredient.

Another advantage is that the form in which the cosmetic patch product is presented does not imply formulations that require the presence of compounds exclusively functional for the structure, since the possible active ingredients are carried and conveyed by the membrane substrate, and the latter is completely biocompatible and absorbable by the skin, without causing any risk or damage to the skin itself.

Another advantage of the cosmetic product lies in the possibility of reaching much higher topical concentrations, for example of hyaluronic acid, than those obtainable with traditional formulations. It is practically impossible to reach high concentrations, even with hyaluronic acid, polysaccharides, collagen, hydroxypropyl methylcellulose HPMC and their low molecular weight derivatives, since the viscosity of the product increases too much and it is not possible to exceed 5-10% by weight. With the present composition, it is possible to obtain cosmetic products that allow to apply concentrations up to 50% by weight of hyaluronic acid on the skin.

According to another aspect, the present invention also concerns a cosmetic article, comprising a cosmetic patch product such as that indicated above disposed on a support base.

Preferably, the cosmetic article comprises a wrapping, which in turn comprises the support base, inside which the cosmetic patch product is present.

Advantageously, the wrapping also comprises a coating layer that acts as protection for the cosmetic patch product present on the support base. The coating layer and the support base are reciprocally joined so as to form an internal space that houses the cosmetic patch product. More preferably, the support base and the coating layer are reciprocally heat-sealed.

Advantageously, the support base and the coating layer are heat-sealable. Preferably, they are made of PBSA (polybutylene succinate adipate) or PLA (polylactic acid).

### BRIEF DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a graphic representation of the evolution of skin hydration with the application of a cosmetic product made with the composition of the invention, and with the application of a placebo product;
- fig. 2 is a graphic representation of the evolution of skin elasticity with the application of a cosmetic product made with the composition of the invention, and with the application of a placebo product;
- fig. 3 is a graphic representation of the evolution of collagen density in skin with the application of a cosmetic product made with the composition of the invention, and with the application of a placebo product;
- figs. 4 and 5 are graphic representations of the evolution of the presence of collagen in skin treated with a cosmetic product made with the composition of the invention, and the comparison with untreated skin.

### DETAILED DESCRIPTION OF SOME EMBODIMENTS

We will now refer in detail to the possible embodiments of the invention. Each example is supplied by way of illustration of the invention and shall not be understood as a limitation thereof. For example, one or more of the characteristics shown or described insomuch as they are part of one embodiment can be varied or adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description. The present description can provide other embodiments and can be obtained or executed in various other ways. We must also clarify that the phraseology and terminology used here is for the purposes of description only, and cannot be considered as limitative.

Unless otherwise defined, all the technical and scientific terms used here and hereafter have the same meaning as commonly understood by a person with ordinary experience in the field of the art to which the present invention belongs. Even if methods and materials similar or equivalent to those described here can be used in practice and in the trials of the present invention, the methods and materials are described hereafter as an example. In the event of conflict, the present application shall prevail, including its definitions. The materials, methods and examples have a purely illustrative purpose and shall not be understood restrictively.

All measurements are carried out, unless otherwise indicated, at 25°C (room temperature) and at atmospheric pressure. All temperatures, unless otherwise indicated, are expressed in degrees Celsius.

All percentages and ratios indicated here are understood to refer to the weight of the total composition (w/w), unless otherwise indicated.

All percentage intervals reported here are supplied with the provision that the sum with respect to the overall composition is 100%, unless otherwise indicated.

All the intervals reported here shall be understood to include the extremes, including those that report an interval "between" two values, unless otherwise indicated.

The present description also includes the intervals that derive from overlapping or uniting two or more intervals described, unless otherwise indicated.

The present description also includes the intervals that can derive from the combination of two or more values taken at different points, unless otherwise indicated.

Where water is mentioned, we mean distilled water, unless otherwise specified.

Embodiments described here concern a cosmetic patch product, preferably to be applied on skin, comprising an electrospun membrane substrate which is made up of at least one electrospun fiber, preferably nanofiber, in the form of non-woven fabric. This form is obtained by progressively depositing the fiber, during the electrospinning, on a support base. The fiber is progressively deposited on several layers which gradually overlap. It is possible to provide more fibers, which can for example be delivered simultaneously on the same support.

The electrospinning occurs by feeding a specific composition under an electric field, through an electrospinning head. The fiber at exit from the electrospinning head is then deposited on a support. The non-woven fabric form can be obtained by means of a relative movement between the electrospinning head and the support.

Preferably, the fiber is continuous, with a homogeneous composition and with a substantially smooth surface. The fiber is also preferably free of defects, that is, free from accumulations or drops of substrate that can form during the electrospinning. Advantageously, the electrospun fiber has a diameter of the order of the nanometer and micrometer, but in any case smaller than 100 µm, more preferably smaller than 50 µm, even more preferably smaller than 25 µm, at most of the order of 10 µm. The fibers of the substrate can have diameters starting from tens of nanometers, for example 50 nm. It should be noted that the diameter remains substantially unchanged along the entire fiber. By substantially unchanged diameter we mean that the diameter can undergo variations which however do not exceed 30% of the average value measured.

Fibers with these diameters have a greater surface/volume ratio than known fibers, which typically have a diameter greater than 100 µm, as well as greater mechanical and structural properties that offer greater efficiency and performance than industry standards. The large specific surface of the fibers obtained makes them particularly suitable to transport and release active ingredients. The nanofibers obtained allow to have a specific surface between 2 and 20 m²/g.

The membrane substrate is made of a biocompatible polymer material suitable to be electrospun selected from a group consisting of polysaccharides, collagen, hydroxypropyl methylcellulose HPMC and their derivatives.

Preferably, the polysaccharide is selected from hyaluronic acid HA, starch, amylose, cellulose, chitosan, glycogen, pectin and agar.

Even more preferably, the substrate comprises linear and/or cross-linked hyaluronic acid. Advantageously, the substrate is made with both linear hyaluronic acid and also cross-linked hyaluronic acid, with linear HA:cross-linked HA proportions ranging from 1:99 to 99:1. Such a mixture allows to modulate the rigidity of the yarn obtained, as well as the three-dimensional structure of the non-woven fabric film.

The hyaluronic acid can have a high mass, for example of the order of a million Dalton or even more, or alternatively have a low mass, typically of the order of 10,000 Dalton or less. The latter form is preferred, since hyaluronic acid with a molecular mass lower than or equal to 10,000 Da, in the electrospun form, once placed in contact with the skin is completely absorbable and is able to penetrate the stratum corneum, reaching the innermost layers where, thanks to its properties such as hygroscopicity, viscoelasticity and structural function, it is able to modulate the hydration of the tissues, the osmotic balance and the physical properties of the ECM.

Advantageously, the action of absorption and penetration into the skin of the membrane substrate also allows to convey possible cosmetic active ingredients associated with the membrane support toward the skin.

In particular, according to some embodiments, the cosmetic product also comprises at least one active ingredient, for example a cosmetic active ingredient, associated with the membrane substrate, preferably selected from polypeptides. The active ingredient is preferably selected from peptides, amino acids, antioxidants and vitamins. In these embodiments, it becomes particularly advantageous to provide that the membrane substrate is made up of a mixture of linear hyaluronic acid with cross-linked hyaluronic acid. The presence of the latter affects the three-dimensional structure of the non-woven fabric, in particular by forming cavities, also called cages, suitable to house the molecules of the active ingredient.

The active ingredient can be added to the already electrospun membrane substrate, therefore in its non-woven fabric form, or it can be included in the composition which is then electrospun. In other words, it is possible to provide that the active ingredient is electrospun together with the material that makes up the substrate.

The present invention also concerns a cosmetic article which comprises the cosmetic patch product described above disposed on a support base. Preferably, the support base forms, together with a coating layer placed so as to cover the cosmetic patch product, a wrapping which then encloses the cosmetic patch product. To this end, the support base and the coating layer are reciprocally joined so as to conform an internal space, intended to house the cosmetic product.

Preferably, the reciprocal joining occurs by means of heat-sealing, even more preferably it is made in correspondence with the edges of the support base and the coating layer. The wrapping is suitable to protect the cosmetic patch product, that is, the membrane substrate and the one or more active ingredients, isolating it from external agents that could damage it or alter its structure and/or chemical composition.

Advantageously, the support base and the coating layer, which can be made of the same material or of two different materials, obviously comprise at least one surface made of a material compatible with the cosmetic product. By compatible with the cosmetic product we mean, in the context of the present description, that the cosmetic product put in contact with this surface is not modified, neither chemically nor structurally. The surface of compatible material does not interact with the substrate or with the active ingredient, but only acts as a support.

Advantageously, the support base and the coating layer are made of a material of the heat-sealable type, for example polybutylene succinate adipate PBSA or polylactic acid PLA.

A preferred method to produce the cosmetic article provides to deposit the electrospun membrane substrate directly on the support base, then to cover the electrospun membrane substrate with the coating layer, and subsequently to join the support base with the coating layer.

As mentioned above, the reciprocal joining of the layers preferably occurs by means of heat-sealing, even more preferably by means of heat-sealing in correspondence with the edges of the support base and the coating layer.

### EXAMPLES

Various compositions, listed below, were electrospun under the conditions indicated. In the composition examples, the first compound, to be electrospun and which therefore makes up the membrane substrate of the cosmetic patch product, is chosen from the compounds listed in the table below:

| | |
|---|---|
| HA1 | linear hyaluronic acid with average molecular mass equal to 1.2 MDa |
| HA2 | hyaluronic acid oligomer with average molecular mass lower than 10000 Da |
| HA3 | hyaluronic acid with average molecular mass equal to 50000 Da |
| HA4 | cross-linked hyaluronic acid with average molecular mass comprised between 20 and 3000 kDa |

These compounds are supplied by Esperis S.p.A., Milan, Evonik Degussa Italia, Cremona, and IRALAB S.p.A., Usmate Velate (MI). The molecular masses were determined by means of GPC (Gel Permeation Chromatography).

The electrospinning was performed in a NANON.01A apparatus of the Japanese company Mecc CO. Ltd. The experimental conditions are indicated in each of the examples below.

The fibers produced were characterized by means of scanning electron microscopy. In particular, they were coated with gold using an EMITECHK950x Turbo Evaporator sputter coater, EBSciences, East Granby, CT, and observed with a Cambridge Stereoscan 440 SEM, Cambridge, UK scanning electron microscope.

### Examples of electrospinning of compositions comprising hyaluronic acid as a compound to be electrospun and a mixture PEO:alginate as a spinning promoter

The spinning promoter comprises a mixture of an aqueous solution of alginate at 5% by weight with an aqueous solution of PEO at 5% by weight in a proportion of 1:1. The promoter was then mixed with an aqueous solution of linear hyaluronic acid with an average molecular weight of 1.2 MDa (HA1) at 0.5% by weight. The promoter:(HA solution) proportion is equal to 5.6:1. The composition was electrospun with relative humidity (RH) comprised between 24% and 29%, at a temperature of 22°C, with an electric field of 20kV, at a volumetric flow rate of the composition at the head equal to 0.7 mL/h, the distance between the spinning head and the support on which the fiber deposits is equal to 15cm and the needle used being a needle with a diameter 22G. The fiber obtained is regular and has few defects. The same promoter was mixed with an aqueous solution of hyaluronic acid oligomer at 13% by weight, in promoter:(HA solution) proportion equal to 1:3. The electrospinning of this second example of composition, under the same operating conditions as the first example above, has a very regular and defect-free fiber, with an average diameter comprised between 250 and 350 nm. The fiber obtained completely covered the support used.

### Examples of electrospinning of compositions comprising hyaluronic acid as a compound to be electrospun and pullulan as a spinning promoter

The pullulan used is of the food grade type, produced by Hayashibara Co., Ltd. Aqueous solutions of pullulan at 10%, 15% or 20% by weight were prepared, and these aqueous solutions of pullulan (spinning promoter) were mixed with aqueous solutions of hyaluronic acid, for the electro spinning.

The table below lists the examples of compositions that were electrospun, as well as the corresponding operating conditions of the electrospinning.

| | Composition | Electrospinning conditions |
|---|---|---|
| 1 | Pullulan 20%:HA3 29% 1:2 | RH 20-30%, 23kV, 0.1µl/min, 15 cm, needle 22G |
| 2 | Pullulan 20%:HA2 29% 1:2 | RH 46%, T=23°C, 23kV, 1ml/h, 15 cm, needle 22G |
| 3 | Pullulan 10%:HA2 23% 1:3 | RH 40%, T=24°C, 23-25kV, 1ml/h, needle 22G, max distance |
| 4 | Pullulan 10%:HA2 15% 1:2 | RH 49%, T=21°C, 23kV, 0.6ml/h, 18 cm, needle 22G, acid pH (between 1.5 and 3) |
| 5 | (pullulan 15%/alginate 5% 3:1):HA2 23% 1:3 | RH 49%, T=21°C, 23kV, 0.6ml/h, 15 cm, needle 22G |
| 6 | Pullulan 10%:HA2 23% 1:3 | RH 49%, T=21°C, 23kV, 0.15ml/h, 15 cm, needle 22G, acid pH (between 1.5 and 3) |
| 7 | Pullulan 10%:HA2 15% 1:2 | RH 40-50%, T=21°C, 23kV, 0.6ml/h, 15 cm, needle 22G, pH = 5.5 |
| 8 | Pullulan 10%:HA2 23% 1:3 | RH 30%, T=22°C, 23kV, 0.5ml/h, 15 cm, needle 22G, pH = 5.5 |

Example 1 resulted in regular fibers, without defects and with an average diameter from 400 to 700 nm. However, little deposit was observed during the test.

In example 2, the fibers obtained are thick, with an average diameter of 10 µm, due to the high viscosity of the electrospun solution.

In example 3, the fibers obtained have an average diameter comprised between 50 nm and 2 µm. It should be observed that with this example the fibers were deposited both on aluminum and also on a film of PBSA.

Examples 4 and 7 (pullulan:HA ratio equal to 1:2), on the one hand, and 6 and 8 (pullulan:HA ratio equal to 1:3), on the other hand, allowed to verify the effect of the proportions between promoter and hyaluronic acid. In example 4, the solution obtained has optimal properties for a good electrospinning, the fibers obtained have an average diameter ranging from 800 nm to 1 µm. For the composition of example 4, which has an acid pH, the pH was increased up to 5.5 (by adding NaOH 1M) thus obtaining the solution of example 7. With the latter, the electrospinning gave regular and uniform fibers with an average diameter smaller than example 4, between 500 and 700 nm.

By increasing the proportion of hyaluronic acid, in example 6 (with acid pH) fibers with a uniform diameter were obtained, with an average value equal to 1-3 µm, while in example 8 (with pH 5.5) the fibers obtained have a non-uniform diameter ranging from 700 nm to 3 µm.

In example 5, an alginate was added to the pullulan as a stabilizer. With a promoter:HA ratio of 1:3, and under the conditions mentioned in the table, thick fibers were obtained, with an average diameter of the order of several microns.

### Preliminary in vivo evaluation of the efficiency of the composition in the cosmetic treatment of skin

A preliminary evaluation was carried out on ten volunteers, who were asked to apply a cosmetic product based on fibers obtained by electrospinning of the composition according to the invention, and a placebo. We hereby wish to clarify that the results set out below are indicative of a technical effect achieved by the composition according to the invention, which however have to be confirmed with further tests.

Each volunteer applied both the cosmetic product and also the placebo, each one on a respective arm, in particular on the palmar zone of the forearm, at the rate of one application per day for a period of four weeks.

The cosmetic product and the placebo were supplied in the form of sheets of electrospun fiber, 4x3 cm in size, on an aluminum support sheet. Both have the same base, and a cosmetic ingredient was added to the cosmetic product.

The cosmetic product used comprises hyaluronic acid HA as first compound to be electrospun, and pullulan as electrospinning promoter. The electrospun solution was prepared by dissolving 25g of hyaluronic acid and 12.5g of pullulan in 30-50mL of water, and taking its volume to 100mL by dilution, checking the pH and possibly correcting it by adding a solution of NaOH 1M so to obtain a pH of 5.5-6.

The placebo solution, on the other hand, only contains pullulan, without hyaluronic acid. Similarly to what described for the cosmetic product, 15gr of pullulan were dissolved in 30-50 mL of water, and taken to 100mL by dilution, checking the pH and possibly correcting it by adding a solution of NaOH 1M in order to obtain a pH of 5.5-6. The solution obtained was then electrospun.

The effects of the treatment were measured using instrumental methods, in particular to assess skin hydration, skin elasticity and collagen density. The measurements were performed before the start of treatment (T0), and after 1 (T7), 2 (T14) and 4 weeks (T28) of treatment.

### Assessment of skin hydration

Skin hydration was measured with a Derma Unit SSC 3 hydration probe from Corneometer^{®}. The probe is constructed with a series of gold metal tracks to function as capacitor plates. The plates are electrically insulated by an electrical insulator, called dielectric. After the connection to an electrical power supply, electrons flow between the plates creating an electric field: the amount of charge stored by the capacitor is called capacitance. Most materials have a dielectric constant greater than vacuum, so that any material whatsoever between the capacitor plates will increase capacitance. The water present in the skin causes a change in capacity proportional to its content, giving a measure of skin hydration in arbitrary units.

### Assessment of skin elasticity

The measurement of skin elasticity was performed with a DermaLab Combo Skinlab elasticity probe, by the company Cortex. The probe is equipped with a chamber in which a vacuum is applied, and which allows to apply a suction on the surface of the skin. The suction method includes a step of elevating and a step of retracting the skin, which are controlled by infrared sensors in the probe chamber. One of the parameters related to skin elasticity is Retraction Time, which represents the time expressed in milliseconds that is necessary for the skin to retract by 1.5 mm, after having been taken to the point of maximum elevation. Young and elastic skin quickly returns to its initial state when it is elevated, while less elastic skin will have longer retraction times. Therefore, a reduction in the retraction time is an indication of an increase in skin elasticity.

### Assessment of collagen density

High resolution images of deeper layers of the skin were obtained with a DermaLab Combo Skinlab (Cortex) ultrasound probe. The technique is based on measuring the acoustic response of the skin, when an acoustic impulse at a known frequency is sent to the skin. This acoustic impulse hits the different structures of the skin and is partly reflected. The part of the signal that is reflected is detected by an ultrasound transducer and processed in order to supply a cross-section image of the skin. The intensity of the reflected signal is indicated with a color scale, in which the darkest zones represent the zones with low response (those in which the density of the structures is low or less), while the lighter areas represent the areas with greater density. This technique allows to reconstruct the image of the skin up to a depth of 3.4 mm with a resolution of 0.06 mm, highlighting the structure of the epidermis, of the dermis and of the subcutaneous layer. An image processing software provides a value of the intensity of the echogenic response, which is directly correlated to the density of the collagen. The energy of the acoustic impulses used is very low and has no negative effect on the skin and on other tissues.

### Conducting the analysis

The measurements were performed in a controlled temperature environment, at 21 ± 2°C, and with a humidity of approximately 60%.

The application of the cosmetic product and of the placebo provides to moisten the skin in correspondence with the zone to be treated, to apply the product sheet with the aluminum surface facing upward, to leave the product to act for 3 minutes on the skin, to remove the aluminum support, and eventually wait for the product to absorb completely, if necessary.

### Results

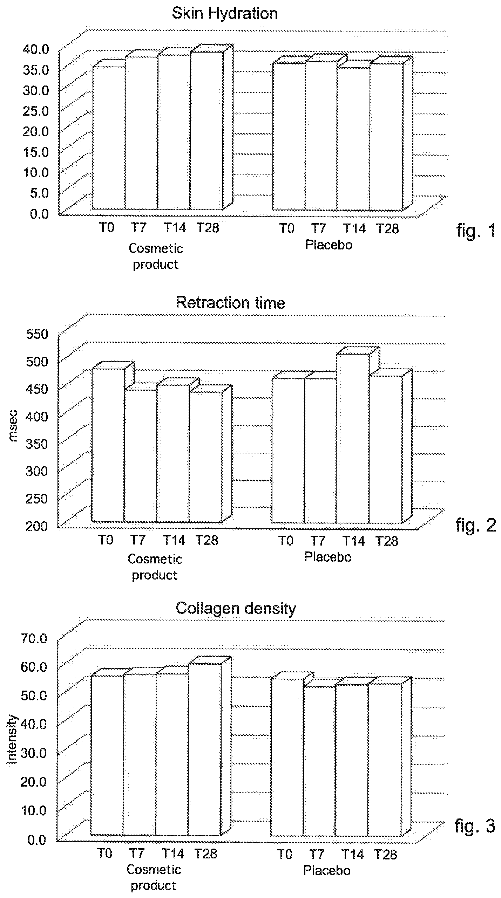

The results of the treatments in terms of skin hydration are summarized in table 1 below and in the graphs in fig. 1. The measurements of skin hydration, expressed in arbitrary corneometric units, are reported as the average value of the values recorded during the test, as the difference and percentage variation between the initial values (T0) and the values recorded at the end of the test, that is, 4 weeks (T28).

| | T0 | T7 | T14 | T28 | Difference T28-T0 | % variation T28-T0 |
|---|---|---|---|---|---|---|
| Cosmetic product | 35.0 | 37.1 | 37.5 | 38.4 | 3.4 | 9.7 |
| Placebo | 36.3 | 36.4 | 35.1 | 36.1 | -0.2 | -0.6 |

The treatment with the cosmetic product showed a significant increase in skin hydration after 28 days. The placebo treatment, on the other hand, showed no significant changes.

The results in terms of skin elasticity are summarized in table 2, and represented in the graphs of fig. 2. The elasticity data, expressed in milliseconds (msec), are reported as the average value of the values recorded during the test, as the difference and percentage variation between the initial values (T0) and the values at the end of the treatment, that is, 4 weeks (T28).

| | T0 | T7 | T14 | T28 | Difference T28-T0 | % variation T28-T0 |
|---|---|---|---|---|---|---|
| Cosmetic product | 483 | 444 | 454 | 441 | -42.0 | -8.7 |
| Placebo | 468 | 468 | 513 | 452 | -16.2 | -3.5 |

For both treatments there was a slight, but not significant, increase in skin elasticity. In the zones treated with the cosmetic product, the increase was greater than in the zones treated with the placebo, however this increase is not significant from a statistical point of view.

The results in terms of collagen density are summarized in table 3 and in the graphs of fig. 3. The collagen density data are reported as the average value of the values recorded during the test, as the difference and percentage variation between the initial values (T0) and the values at the end of the treatment, that is, 4 weeks (T28).

| | T0 | T7 | T14 | T28 | Difference T28-T0 | % variation T28-T0 |
|---|---|---|---|---|---|---|
| Cosmetic product | 56.1 | 56.1 | 56.4 | 60.3 | 4.1 | 7.4 |
| Placebo | 55.8 | 52.9 | 53.6 | 53.8 | -2.0 | -3.6 |

The treatment with the cosmetic product allowed to find an increase in collagen density, however this increase is not statistically significant. On the other hand, no increase was measured in the treatment with placebo.

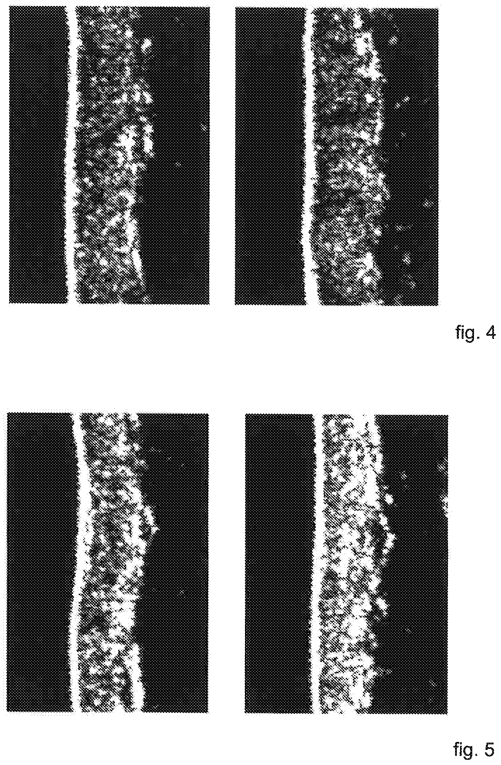

Another study was conducted to evaluate the effect of the cosmetic product on collagen density. The application modes of the cosmetic product were the same as those indicated above, but the treatment was carried out over 8 weeks. Figs. 4 and 5 represent a cross-section image of the skin, processed starting from the data collected by the instrument used, indicated above. The white zones indicate the presence of collagen. Fig. 4 shows the section of an untreated zone of the skin, at the beginning of the treatment (T0, to the left) and after 8 weeks of treatment (to the right). Fig. 5 instead shows a treated zone of the skin, at the beginning of the treatment (T0, to the left) and at the end of the treatment after 8 weeks (to the right).

In the treated zone there is a visible increase of collagen in the skin at the end of the treatment, this increase not being found in the untreated zone.

In conclusion, the tests described above offer a preliminary evaluation of the efficiency of the cosmetic product compared to a placebo. It was possible to observe a tendency toward improvement, for all three parameters monitored, which was greater with the cosmetic product than with the placebo.

The tests carried out allowed to detect a perceptible tendency toward improvement, at least as regards collagen density.

This tendency is, among other things, confirmed by the last collagen density test, in which application times were doubled (eight weeks instead of four). The evolution of collagen density is even more evident, even if only from a visual verification. It should be emphasized that the improvement observed in the status of the collagen fibers in the skin was obtained with a single daily application of the cosmetic product, applying only hyaluronic acid, and in a shorter time than with cosmetic products of the state of the art. By way of comparison, with known products, obtaining an improvement of the collagen fibers in the skin requires longer application times (at least three months) and a greater number of daily applications.

In light of the results obtained from the preliminary study described above, it is possible to observe an interesting tendency toward improvement of the zone treated with the cosmetic product compared to the placebo, for the parameters measured, in particular for hydration and collagen density.

## Claims

1. Cosmetic patch product comprising a membrane substrate suitable to be absorbed by the skin, said membrane substrate being made up of electrospun fibers made up of hyaluronic acid and an electrospinning promoter selected from pullulan or a mixture of alginate with poly(oxyethylene).

2. Product as in claim 1, **characterized in that** the membrane substrate comprises at least one electrospun fiber and is in the form of non-woven fabric.

3. Product as in claim 2, **characterized in that** the fiber has an average diameter of less than 100 µm.

4. Product as in any claim hereinbefore, **characterized in that** said membrane substrate comprises at least one active ingredient.

5. Product as in claim 1, **characterized in that** the hyaluronic acid has a molecular mass of less than 10000 Da, measured by gel permeation chromatography as described in the description.

6. Product as in any claim hereinbefore, **characterized in that** the membrane substrate is made in a mixture of linear hyaluronic acid with cross-linked hyaluronic acid.

7. Cosmetic article, comprising a cosmetic patch product as in any claim hereinbefore, disposed on a support base.

8. Cosmetic article as in claim 7, **characterized in that** it comprises a wrapping inside which said cosmetic patch product is present, said wrapping comprising said support base.

9. Cosmetic article as in claim 7 or 8, **characterized in that** said support base is made of PBSA or PLA.

10. Cosmetic article as in claim 8, or as in claim 9 when it depends on claim 8, **characterized in that** the wrapping also comprises a coating layer which acts as protection for said cosmetic patch product present on said support base, wherein said coating layer and said support base are reciprocally joined so as to form an internal space which houses the cosmetic patch product.

11. Cosmetic article as in claim 10, **characterized in that** said coating layer is made of PBSA or PLA.

## Patentansprüche

1. Kosmetisches Pflasterprodukt, umfassend ein Membransubstrat, das zur Absorption durch die Haut geeignet ist, wobei das Membransubstrat aus elektrogesponnenen Fasern zusammengesetzt ist, die aus Hyaluronsäure und einem Elektrospinning-Promotor, der ausgewählt ist aus Pullulan oder einer Mischung aus Alginat mit Poly(oxyethylen), zusammengesetzt sind.

2. Produkt nach Anspruch 1, **dadurch gekennzeichnet, dass** das Membransubstrat wenigstens eine elektrogesponnene Faser umfasst und die Form eines Vliesstoffs aufweist.

3. Produkt nach Anspruch 2, **dadurch gekennzeichnet, dass** die Faser einen Durchmesser von weniger als 100 µm aufweist.

4. Produkt nach einem der vorstehenden Ansprüche, **dadurch gekennzeichnet, dass** das Membransubstrat mindestens einen Wirkstoff umfasst.

5. Produkt nach Anspruch 1, **dadurch gekennzeichnet, dass** die Hyaluronsäure eine Molekülmasse von weniger als 10000 Da, gemessen mittels Gelpermeationschromatographie wie in der Beschreibung beschrieben, aufweist.

6. Produkt nach einem der vorstehenden Ansprüche, **dadurch gekennzeichnet, dass** das Membransubstrat aus einer Mischung aus linearer Hyaluronsäure und vernetzter Hyaluronsäure hergestellt ist.

7. Kosmetikartikel umfassend ein kosmetisches Pflasterprodukt nach einem der vorstehenden Ansprüchen, das auf einer Trägerbasis angeordnet ist.

8. Kosmetikartikel nach Anspruch 7, **dadurch gekennzeichnet, dass** er eine Umhüllung umfasst, in welcher sich das kosmetische Pflasterprodukt befindet, wobei die Umhüllung die Trägerbasis umfasst.

9. Kosmetikartikel nach Anspruch 7 oder 8, **dadurch gekennzeichnet, dass** die Trägerbasis aus PBSA oder PLA hergestellt ist.

10. Kosmetikartikel nach Anspruch 8, oder nach Anspruch 9, wenn dieser von Anspruch 8 abhängig ist, **dadurch gekennzeichnet, dass** die Umhüllung auch eine Beschichtungsschicht umfasst, die als Schutz für das auf der Trägerbasis angeordnete kosmetische Pflasterprodukt dient, wobei die Beschichtungsschicht und die Trägerbasis wechselseitig miteinander verbunden sind, um einen Innenraum zu bilden, in dem das kosmetische Pflasterprodukt aufgenommen ist.

11. Kosmetikartikel nach Anspruch 10, **dadurch gekennzeichnet, dass** die Beschichtungsschicht aus PBSA oder PLA hergestellt ist.

## Revendications

1. Produit de patch cosmétique comprenant un substrat membranaire approprié pour être absorbé par la peau, ledit substrat membranaire étant composé de fibres électrofilées composées d'acide hyaluronique et d'un promoteur d'électrofilage sélectionné parmi le pullulane ou un mélange d'alginate avec du poly(oxyéthylène).

2. Produit selon la revendication 1, **caractérisé en ce que** le substrat membranaire comprend au moins une fibre électrofilée et se présente sous forme de tissu non tissé.

3. Produit selon la revendication 2, **caractérisé en ce que** la fibre présente un diamètre moyen inférieur à 100 µm.

4. Produit selon une quelconque revendication précédente, **caractérisé en ce que** ledit substrat membranaire comprend au moins une ingrédient actif.

5. Produit selon la revendication 1, **caractérisé en ce que** l'acide hyaluronique présente une masse moléculaire inférieure à 10000 Da, mesurée par chromatographie par perméation sur gel comme décrit dans la description.

6. Produit selon une quelconque revendication précédente, **caractérisé en ce que** le substrat membranaire est préparé dans un mélange d'acide hyaluronique linéaire avec de l'acide hyaluronique réticulé.

7. Article cosmétique, comprenant un produit de patch cosmétique selon une quelconque revendication précédente, disposé sur une base de support.

8. Article cosmétique selon la revendication 7, **caractérisé en ce qu'**il comprend un emballage à l'intérieur duquel est présent ledit produit de patch cosmétique, ledit emballage comprenant ladite base support.

9. Article cosmétique selon la revendication 7 ou 8, **caractérisé en ce que** ladite base de support est en PBSA ou en PLA.

10. Article cosmétique selon la revendication 8, ou selon la revendication 9 lorsqu'elle dépend de la revendication 8, **caractérisé en ce que** l'emballage comprend également une couche de revêtement qui sert de protection pour ledit produit de patch cosmétique présent sur ladite base de support, dans lequel ladite couche de revêtement et ladite base de support sont assemblées l'une avec l'autre de manière à former un espace interne qui loge le produit de patch cosmétique.

11. Article cosmétique selon la revendication 10, **caractérisé en ce que** ladite couche de revêtement est en PBSA ou en PLA.
